Europäisches Patentamt

European Patent Office

Office européen des brevets

(11) Publication number: **0 394 788 B1**

# EUROPEAN PATENT SPECIFICATION

(45) Date of publication of patent specification: **03.08.94** (51) Int. Cl.5: **C12N 5/00**, C12N 1/04, C12M 3/00

(21) Application number: **90107137.3**

(22) Date of filing: **13.04.90**

(54) **Large scale fermenter inoculation with frozen cells.**

(30) Priority: **28.04.89 US 344730**

(43) Date of publication of application:
**31.10.90 Bulletin 90/44**

(45) Publication of the grant of the patent:
**03.08.94 Bulletin 94/31**

(84) Designated Contracting States:
**AT BE CH DE DK ES FR GB GR IT LI LU NL SE**

(56) References cited:
**DD-A- 140 476**

**J. of Clinical Pathology, vol. 34, 1981, London, GB; A. TAYLOR, "cryopreservation of platlets: an in-vitro comparison of four methods", pp. 71-75**

(73) Proprietor: **MILES INC.**
**Fourth and Parker Streets**
**P.O. Box 1986**
**Berkeley California 94701(US)**

(72) Inventor: **Schwartz, Barry D.**
**254 Rutherford Avenue**
**Redwood City, California 94061(US)**
Inventor: **Trawinski, Jurgen**
**3412 Maricopa**
**Richmond, California 94084(US)**

(74) Representative: **Dänner, Klaus, Dr. et al**
**Bayer AG**
**Konzernverwaltung RP**
**Patente Konzern**
**D-51368 Leverkusen (DE)**

Rank Xerox (UK) Business Services
(3.10/3.09/3.3.3)

## Description

### 1. Field of the Invention:

The present invention relates to the cryopreservation of mammalian cells, and more particularly to the use of mammalian cells in tissue culture, wherein the cells are maintained frozen prior to bioreactor inoculation.

### 2. Description of the Related Art:

Mammalian cells are cultured in large scale for the production of vaccines, monoclonal antibodies, recombinant DNA products and other biologicals. Numerous large scale production systems are known. These may involve the use of deep tank fermenters, hollow fibers, ceramic matrices, or other types of bioreactors. Typically, commercial scale production of products from mammalian cells requires the use of large numbers of cells in bioreactors having at least 50 liters of working volume. Deep tank fermenters in the range of 1,000 liters or greater are used for commercial production.

The mammalian cells which are used in the large scale production of biologicals are stored frozen in ampules which contain, typically, 2-6 million cells dispensed into 1 ml aliquots. (See, ATCC Quality Control Methods for Cell Lines, 1st ed., 1965, R. Hay, ed.) These ampules constitute a frozen cell stock which is stored as a master cell bank (MCB) and/or a manufacturer's working cell bank (MWCB). The use of such an MCB or MWCB is recommended by regulatory authorities. See, Points to Consider in the Characterization of Cell Lines Used to Produce Biologicals (1987), Office of Biologics Research and Review, U.S. Food and Drug Administration. These "Points to Consider" further endorse the practice of culturing mammalian cells at defined population doubling levels (PDL). In the case of a continuous cell line, the MCB should be cloned, i.e. derived from a single cell. The ampules are filled with cells at a low PDL and stored at -196° C in liquid nitrogen. When a bioreactor inoculum is needed, ampules are thawed and the cells are expanded. The method used to expand the cells is dependent on the requirements for the particular cell line. For example, some lines are anchorage- dependent, whereas others grow in suspension; and some cells have unusual nutritional requirements in the early expansion phase. Expansion is carried out by first thawing the cells and growing them in a small tissue culture flask. Cells from this flask are diluted and placed into a larger flask or two small flasks; this process is repeated until the requisite number of cells has been obtained for inoculation into a large scale bioreactor. Because this is a time-consuming process, the only way to assure a continuous fermentation is to constantly keep cells in small-scale culture and hold them ready at all times for reinoculation of the bioreactor in the event of contamination or other technical failure. The time needed to expand the cells as well as the costs involved in the small-scale cultivation could be greatly reduced by freezing and stockpiling enough cells for one inoculation of the production system in one aliquot.

Mammalian cells are typically put up into cell banks in ampules containing the appropriate culture medium plus 5-10% (by volume) glycerol or DMSO. ATCC recommends that the ampules be cooled at 1-3° C per minute down to -30° C. At -30° C, the ampules should be cooled rapidly to -150° C and transferred to liquid nitrogen.

Other freezing rates and cryoprotective agents for freezing cultured mammalian cells lines in ampules are known. See Klebe et al., "Identification of New Cryoprotective Agents for Cultured Mammalian Cells," In Vitro 19(3):167-170 (1983). DMSO is the most commonly used cryopreservative compound. A review of the various uses of DMSO is found at JAMA Sept. 17, 1982 248(11):1369-1371.

Cryopreservation has been used in other fields besides the field of culturing mammalian cell lines. For example, Rall, "Factors Affecting the Survival of Mouse Embryos Cryopreserved by Vitrification," Cryobiology 24:387-402 (1987) describes the preservation of 8 cell mouse embryos by cooling in a vitrification solution, which dehydrates the embryos, followed by storage in liquid nitrogen. Lawrence et al. U.S. Patent No. 4,155,331 describes the cryopreservation of shrimp, using a rapid cooling rate. A cryopreservative is used and the materials may be stored at -70° C or at temperatures as low as -200° C.

Cryopreservation of human platelets is also used to store these cells for subsequent therapeutic administration. Spector et al., "Viability and Function of Platelets Frozen at 2 to 3° Per Minute with 4 or 5 Per Cent DMSO and Stored at -80° C for 8 Months," Transfusion 17(1):8-14 (1977), describe the cryopreservation of 50 ml platelet concentrates by the addition thereto of 50 ml 8% DMSO in autologous plasma and frozen in a 200 cm$^2$ surface area bag at -80° C. Taylor, "Cryopreservation of platelets: an in vitro comparison of four methods," J. Clin. Path. 34:71-75 (1981), describes four different cryopreservative solutions; 5% DMSO was the most suitable solution, even though many platelets (>50%) are lost with use of this solution. The platelets were frozen in polyolefin platelet freezing bags, with controlled rate cooling and storage in liquid nitrogen. Schiffer et al., "A Clinical Program of Platelet Cryopreservation," Cytaphereis and Plasma Exchange:Clinical Indications, pp. 165-180 (Alan R. Liss, Inc. 1982), describe a program of platelet cryopreservation used at Baltimore Cancer Research Center. Between 4-6 units of platelets (3-4.5 x $10^{11}$ platelets) are concentrated and resuspended in 50 ml of plasma and transferred to a 200 ml polyolefin bag to which 50 ml of 10% DMSO-autologous plasma is added. The units are stored at -80°C. Storage at -120°C is reported. Platelets are generally stored at -90°C to -120°C, with DMSO as a cryopreservative.

Also, human monocytes have been separated from peripheral blood and stored at -195°C for later infusion. 0.8 ml aliquots of a suspension of monocytes were frozen in Hank's Balanced Salt Solution containing 20.5% DMSO, 15.5% acetamide, 10% propylene glycol and 6% polyethylene glycol.

Cryopreservation of human bone marrow cells is also described in the literature. Yeager et al., "Autologous Bone Marrow Transplantation in Patients with Acute Nonlymphocytic Leukemia, Using Ex Vivo Marrow Treatment with 4- Hydroperoxycyclophosphamide," N. Engl. J. Med 315(3):141-147 (1986), describes bone marrow that had been collected during remission, cryopreserved, and treated with 4-hydroperoxyclophashamide alkylating agent prior to transplantation. 4-6 x $10^8$ nucleated bone marrow cells per kg body weight were collected. The nucleated buffy coat was separated and mixed with autologous plasma and heparinized tissue culture medium (TC 199) to obtain a concentration of 2 x $10^7$ cells per ml. The cells were treated with the alkylating agent, centrifuged, resuspended in 45% plasma, 45% tissue culture fluid, and 10% DMSO at a concentration of 4 x $10^7$ cells per ml 50 ml aliquots of the cell suspension were placed in polyolefin bags and frozen in liquid nitrogen.

Beujean et al., "Successful Infusion of 40 Cryopreserved Autologous Bone-Marrows," Biomedine and Pharmacotherapy, 38:348-352 (1984), describe a cryopreservation protocol in which large amounts of bone marrow are removed from the donor/recipient. The marrow was mixed with a cryoprotective solution consisting of 20% DMSO and 10% matched human serum in TC 199. Aliquots of 200 ml were transferred to polyolefin freezing bags and cooled in a programmed freezing schedule to -196°C.

Dovay et al., "A Technical Bias: Differences in Cooling Rates Prevent Ampoules from Being a Reliable Index of Stem Cell Cryopreservation in Large Volumes," Cryobiology 23:296-301 (1986), describes the survival of bone marrow progenitor cells (CFU-GM) in ampules versus bags. The marrow was mixed with an equal volume of tissue culture fluid containing TC 199, 20% DMSO and 10% matched serum. Freezing was carried out at a controlled rate, and the bags were stored in liquid nitrogen.

Summary of the Invention

A method is described for the large-scale freezing of mammalian cells in flexible bags and their use for the direct inoculation of bioreactors (e.g. fermenters) to be used in the production of commercially valuable cellular proteins and antibodies. Cell suspensions from roller bottles, flasks or fermenters may be concentrated by centrifugation, filtration, or cell settling and treated with the cryopreservative dimethyl sulfoxide (DMSO) at a final concentration of 5-15% by volume. The cells are distributed among 100-ml or 500-ml preservation bags, frozen in liquid nitrogen vapor, and stored at -70° (dry ice) or -196°C (liquid nitrogen). Storage of at least 9 months at -70°C or below was possible without cellular deterioration. Cell inocula for fermenter runs are prepared by removing a frozen bag from the freezer and thawing the contents rapidly at 37°C in a water bath. The thawed suspension is connected to a fermenter inlet line and directly infused into the system without any need for a pre-culture period. Because of the high density and high volume of cells in the blood bag, it is possible to inoculate a 15-L fermenter with the contents of a single 500-ml bag and obtain an inoculation density in the fermenter of 1 x $10^6$ viable cells per ml. In the case of cells such as engineered BHK cells which make recombinant Factor VIII:C protein (termed herein, "F8 cells"), this starting density is critical since it is sufficient to start the fermenter immediately in a production mode. Among the benefits produced by the large-scale cell freezing technique are the following: substantial savings of time and money due to elimination of scale-up of the inoculum; rapid recovery from fermenter losses caused by contamination and technical malfunctions; increased homogeneity of fermenter inocula; extended storage capability of production quality inocula; and the possibility of transporting high quality seed stocks to any production facility in the world.

It is possible to freeze genetically modified BHK cells and monoclonal-antibody producing lymphoid lines in 500-ml aliquots at densities equal to 1 x $10^7$ to 4.5 x $10^7$ cells per ml. The cells are cryopreserved in dimethyl sulfoxide (DMSO), and controlled-rate freezing is not necessary. According to the present invention, fermenters larger than 15 L may be inoculated by multiple 500 ml bags or by larger bags. Bags of unlimited size could be used, provided that the thickness of the bag is maintained between approximately .6 cm to .85 cm. By "approximately," it is expected that ± 20% variability in thickness could be tolerated.

The practicality and value of this technique is supported by the fermenter runs described below. Our studies have shown that the cell inocula derived from the frozen bag technique are at least as good as, if not better than, the inocula derived from the standard seed-train method. With the cell bag technique, significant quantities of Factor VIII secreted from F8 cells are reported in the fermenters in as little as 3 days after inoculation. This and other improvements in cell growth and productivity may be consequences of the fact that the frozen cells were derived from fermenter-adapted cell populations and not laboratory cell lines. The cells were in an active state of proliferation and product secretion when frozen and were already adapted to serum-free conditions. It appears that the thawed population retains all of these desirable characteristics. It is possible that the continued adaptation and selection of large-scale fermenter cell populations, now feasible because of the large-scale freezing approach, may yield further improvements in culture productivity and the development of uniquely specialized fermenter cell lines.

Another important aspect of the present invention is the immediate productivity of the cells upon inoculation into the fermenter. Inoculation results in high cell viability (>70%) due to freezing conditions, including the use of DMSO. The DMSO is diluted to less than 1% volume/volume concentration in the fermenter.

For purposes of the present invention, the following definitions are used:

A fermenter, or a bioreactor, is a vessel containing hardware for the controlled flow of gases and nutrients therethrough, as opposed to a roller bottle or tissue culture flask. Fermentation technology is well known in various configurations, such as deep tank, hollow fiber, ceramic cartridge, microcarrier, encapsulation, etc.

Cell density is mean viable cell number per ml of culture fluid.

An immortal cell is one which can undergo an unlimited number of cell divisions, as opposed to a primary cell, which normally will undergo a finite number of divisions in culture. Examples of immortal cells (cell lines) are BHK-21 cells, CHO cells, hybridomas, EBV-transformed lymphocytes, etc.

Viability is measured by the trypan blue dye exclusion test.

Flexible film is considered herein to be a plastic sheet material having a thickness of 0.010 to 0.090 mm.

## Brief Description of the Drawing

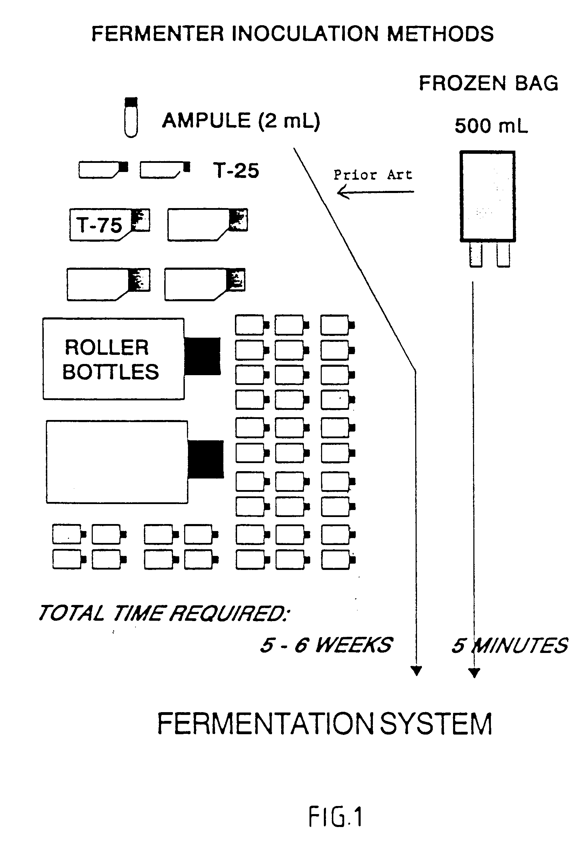

Fig. 1 is a schematic representation of the present invention as compared to the prior art; and

Fig. 2 is a graph showing optimum cell density for freezing, as determined by experiments in 2 ml ampules, with BC cells and 10% DMSO.

## Description of the Preferred Embodiment

### 1. Materials and Methods

#### Cell cultures used for freezing

The cell lines used in these experiments were the proprietary BHK lines used for recombinant Factor VIII production, (Nature, 312:330-337(1984), EPO 160 457) and the proprietary line BC, for anti-Pseudomonas monoclonal antibody production. The BC cells were prepared from EBV-transformed human lymphocytes. A technique to produce such cells is described in U.S. 4,446,465. Cultures were initiated from MWCB material, and experiments were conducted with cells at 21 to 48 population doubling levels (PDL's) (16 to 32 passages) from the source material. The F8 cells were taken from roller bottles or directly from cell suspensions harvested from fermenters used in the clinical production of Factor VIII.

#### Culture media

All culture media were proprietary formulations produced at the Cutter-Berkeley site. The BC cells were cultivated in serum-free MOAB complete medium. F8 cells in roller bottles were carried in growth medium (G), whereas fermenter cells used Production Medium (PM) in conjunction with dialysis medium (DM). MOAB medium consisted of modified Dulbecco's Eagles' medium (DMEM)/Ham's F-12 medium (F-12) in a 1:1 ratio plus 10 mg/L insulin 11 mg/L transferrin. 5 mg/L oleic acid, 1 mg/L cholesterol and 10 ml/L 20% human albumin.

GM consisted of DMEM/F-12, 1:4 ratio, without glucose, thymidine, glycine, hypoxanthine, plus sodium pyruvate 0.110 g/L, glutamine 0.730 g/L, Hepes buffer 15 mM, mannose, 2 g/L, methotrexate 1.5 mg/L, NaHCO$_3$ 1.2 g/L dialyzed fetal bovine serum 50 ml/L and 10 mg/L insulin.

PM consisted of DMEM/F-12, 1:1 ratio, with glucose 1g/L, mannose 3 g/L. ethanolamine 1.2 mg/L, phosphoethanolamine, 1.4 mg/L, d-biotin 1 mg/L. glutathione 1 mg/L, mercapto ethanol 5 $\mu$M, Se0$_2$ 20 nM, MEM vitamins, [100 x] 1%, MEM amino acids [100 x] 2% MEM non-essential amino acids [100 x] 1%, glutamine 5 mM, insulin, 10mg/L, oleic acid 1 mg/L, cholesterol 0.1 mg/L and 5% plasmanate PPF 60 ml/L. DM consisted of DMEM/F-12, 1:1, with glucose 1g/L, mannose 3 g/L, ethanolamine 1.2 mg/L, phosphoethanolamine 1.4 mg/L, d-biotin 1 mg/L, glutathione 1 mg/L, Mercapto ethanol 5 $\mu$M, Se0$_2$ 20 nM, MEM vitamins [100 x] 1%, MEM amino acids [100 x] 2%, MEM non-essential amino acids [100 x] 1%, glutamine 5 mM, MgCl$_2$ 3.05 g/L, NaHC0$_3$ 2 g/L.

Cryopreservation media

The cryopreservative of choice was 10% dimethylsulfoxide (DMSO) (Sigma Chemical Company, St. Louis, MO) in PM or MOAB medium. When F8 cells were used, the medium was composed of 25-50% Plasmanate (Cutter Biological, Berkeley, CA), a 5% plasma protein fraction (PPF). The net PPF concentration was therefore approximately 1%-2%. In addition, some experiments were conducted with 10% glycerol (Sigma) in MOAB medium.

Culture Vessels and Conditions

Some cells were grown in 850-cm$^2$ roller bottles (Falcon, Becton-Dickinson & Co., Lincoln Park, NJ). The 15-L working volume fermenters used were made by either the Virtis Co., Inc. (Gardiner, NY) or by Chemap Inc., USA (South Plainfield, NJ). Roller bottles were maintained at pH 6.8 to 7.2 and split twice weekly at a split ratio of either 1:2 or 1:4. Incubation was in tightly closed containers at 37°C. F8 cultures were rotated at a speed of 8 RPM; BC cells were rotated at 4RPM.

Containers for cryopreservation

Plastic ampules (2-ml) were obtained from Corning Glass Works (Corning, NY). Bone marrow freezing bags (100 ml) and red blood cell freezing bags (500 ml) were obtained from Stericon Inc. (Broadview, IL). In addition, 500-ml freezing bags capable of withstanding liquid nitrogen temperature were obtained in collaboration with Cutter's Departments of Blood Bag Technology and Blood Bag Systems Research. These bags were composed of EVA and had a film thickness of .02-.04 mm. Viable cell recovery and viability ratios were superior to the Stericon bone marrow bags.

2. General Procedure

In general, a healthy cell suspension from roller bottles or fermenters was first collected in a holding vessel, either a 3-L spinner flask or a 20-L polypropylene jug. Cells more than 5 x 10$^6$ cells per ml in density were immediately diluted 1:1 with growth medium (PM or MOAB medium) to provide nutrients and forestall acidification during subsequent processing. Up to 10 L of fermenter suspension were collected in this manner.

The following general protocol was observed:

1. Collect cells from roller bottles or fermenters.
2. Pump cells into holding vessel for dilution and stirring.
3. Transport apparatus and cells into cold room (4°C) or chill to 4°C in ice bath.
4. Concentrate cell suspension using continuous centrifuge, hollow fiber cartridge or 1 g settling.
5. Add cryopreservative solution to concentrated cells.
6. Distribute cell suspension to bags.
7. Weigh and seal bags and place them inside metal cassettes.
8. Freeze in liquid nitrogen vapor or in a mechanical freezer at -70°C.
9. Store until needed.

In early experiments, cells were taken directly from the holding vessel and concentrated with the aid of a standard laboratory centrifuge. Due to the limited volume of cell suspension that could be processed in this way, this approach was eventually abandoned in favor of hollow-fiber filtration or continuous centrifugation to concentrate the cell suspension prior to freezing. In these cases, the concentration of the cells and distribution into freezing bags was carried out at 4°C.

Concentration of cells reduces storage space and facilitates fermenter reseeding logistics. The most successful hollow fiber unit for cell concentration was the Plasmapur I cartridge (0.6 um pore size) (Organon Teknika B.V., Boxtel, Holland). The continuous centrifuge used was made by the Haemonetics Corporation (Braintree, MA). Haemonetics cell separation devices have been reported previously in the literature. A general description of this type of apparatus is given in U.S. 4,285,464. Gravity separation has also been successfully used.

Using any one of these methods, the viable cell number was concentrated to 1 to $3 \times 10^7$ cells per ml. For F8 cells, a concentrated cryopreservative solution consisting of 50% DMSO and 25% PPF in a base of either MOAB medium or PM was carefully added to the concentrate at a rate of approximately 100 ml/min. (In one F8 experiment, 1:1 DMSO:PPF was used.) The cryopreservative solution used for the BC cells did not contain PPF. After mixing, the final concentrations of DMSO and PPF were 10% and 5%, respectively. Although different DMSO incubation temperatures and times were investigated, there appeared to be no significant barrier to cellular penetration of DMSO, even at 4°C. Consequently, the cell concentrate was immediately distributed to freezing bags as soon as the cryopreservative was well mixed with the cell suspension.

Blood storage bags of different sizes from various manufacturers were used. For freezing and storage at liquid nitrogen temperatures, the 100-ml Stericon Bone Marrow bag (RC-91F), as described in U.S. 4,468,227, and a specially made 500-ml Cutter EVA bag were used. For short term freezing and storage at -70°C, we used the 500-ml Stericon Platelet Bag (RC-3). An autoclavable 200 ml sample bag from Cutter (product code 20-1528) was used to collect 30-50 ml of cell concentrate at the end of the distribution step. This sample was counted to provide the baseline data later used to determine viable cell recoveries. An electronic scale was used to measure indirectly the volume of the suspension in the bags being filled (one gram was considered equal to one ml for bag-filling purposes).

The cell bags with cryopreservative remained at 4°C for approximately 15-30 minutes during the various processing steps. They were sealed with an impulse heater (Stericon) and placed inside metal cassettes (Stericon). Freezing of the cell suspension was achieved by placing the cassettes horizontally in liquid nitrogen vapor overnight. No programmed freezing unit was used except for one experiment. Those bags that were unable to withstand liquid nitrogen temperatures were placed horizontally in metal cassettes and put on shelves of a mechanical freezer (-70 to -80°C) overnight. All cassettes were maintained continuously in the cold until use.

It is important that the bags be laid flat so that the thickness of the bag is approximately 0.6-0.9 cm. This prevents freezing damage to cells in the center of a thick container.


3. Special Procedures to Increase Cell Density


Achieving high cell densities ($1 \times 10^7$ viable cells per ml or greater) is essential to the success and practicality of this technique. It is also one of the major technical pitfalls, because of the unconcentrated starting material. Three methods for accomplishing high density are given below.

In the first method, cells are grown to extremely high densities within a stirred-tank fermenter and then harvested directly after addition of cryoprotectant solution. No other device for cell concentration is required. Such a procedure may be outlined as follows:

1. Grow fermenter suspension to a viable cell density of approximately $3 \times 10^7$ cells per ml. Alternatively, concentrate cells from production fermenters by cell settling after cooling the fermenter suspension.

2. Add DMSO solution slowly to cell suspension within fermenter. Make certain fermenter system has been cooled to approximately 4°C before adding DMSO.

3. Reverse pump and distribute cell suspension to bags.

4. Weigh, seal, freeze, and store.

The details of the procedure used in the current study were as follows: F8 cells were grown in a 15-L Virtis fermenter in MOAB complete and dialysis media (Expt. FR15). The inoculum for this fermenter had been derived from a 500-ml Stericon bag that had itself been created during an earlier freezing experiment (FR10-2); these cells had been stored for 99 days at -70°C before being used to inoculate a new fermenter at $1 \times 10^6$ viable cells per ml. The cells grew to a density of $2.8 \times 10^7$ viable cells per ml (at 92% viability) within 13 days of culture before being harvested for cryogenic preservation. The 15 L of cell suspension present in the fermenter was then drained to 10 L due to volumetric considerations. Cryopreservative solution (2.5 L) (50% DMSO, 25% PPF, in MOAB complete medium) was added at a speed of 90-130 ml/min directly to 10 L of fermenter suspension and mixed by the system impeller to a final concentration of 15% DMSO and 5% PPF. (The fermenter temperature was left at 37°C during this procedure.) As soon as all of the DMSO had been added, the pump was reversed and distribution to 500-ml blood bags began. Fifteen bags were filled, weighed, sealed, logged and frozen. The entire procedure required 98 min of processing time starting from the addition of DMSO to the freezing of the bags. Cell bags were stored in liquid nitrogen until use as inocula in thawing experiments.

In the second method, a fermenter suspension is concentrated by gravity sedimentation in the fermenter or in an appropriate external vessel. The suspension is first cooled to 4°C to lower cell metabolism, and all pumps and impellers are turned off. The cells are allowed to settle by gravity to the bottom region of the vessel for an appropriate length of time, usually 30 minutes to an hour, and the cell-depleted supernatant is discarded. The resultant cell concentrate is then mixed with cryoprotectant solution and dispensed into freezing bags.

As a third method, it might be necessary to concentrate a dilute fermenter suspension by filtration, or some other physical method that separates the cells from the bulk of their spent medium.

### 4. Reconstitution of Frozen Cell Cultures

Frozen cell suspensions were rapidly thawed by immersion and agitation within a 37°C water bath. Ampules were placed directly in the water bath after removal from the liquid nitrogen freezer; in the case of the bags, the metal cassettes were first removed before bag immersion. The ampules and bags were thawed in 2-4 minutes and were then carefully blotted dry. The suspension within the bags was manipulated during this procedure so as to dissociate cell clumps that might have formed.

The plastic covering of the bag outlet port was swabbed with 70% ethanol and dried. The preparation was then transported to a laminar flow hood, and standard blood-bag spikes with attached sterile tubing were used to open the outlet port. The cell suspension was drained or pumped into a holding vessel and thoroughly mixed. Samples were taken for cell counts and pH determinations. Once the viable cell density of the thawed cell suspension was known, the cell inoculum was calculated and placed in roller bottles or fermenters.

The success or failure of a given freezing experiment was first estimated by determination of the viable cell recovery, i.e. the number of viable cells per ml found in the thawed cell suspension divided by the number of viable cells per ml frozen down. Expressed as a percentage, this quantity will here be called the percent viable cell recovery (% vcr). The quality of the freeze/thawed suspension was also judged by standard percent cell viability determinations. A "viability ratio" was routinely calculated by dividing the percent viability of the thawed cell population by that of the original starting population at the time of cell freezing. Finally, in some cases, thawed cells were tested by cultivation in flasks or roller bottles before actual fermenter experiments were performed.

### 5. Determination of Optimum Cell Density for Freezing

The following experiments were conducted in order to determine the maximum cell density that could be used for cryopreservation without a major loss in cell viability. A suspension of BC cells was concentrated by centrifugation into a pellet which was used to generate a series of eight 2-ml ampules representing serial two-fold dilutions of the pellet. Cells from each dilution were frozen in culture medium containing 10% DMSO. After storage at -70°C for 26 hr and -196°C for 30 hr, the ampules were systematically thawed in a water bath at 37°C with constant agitation. The thaw time for each ampule was approximately 100 seconds.

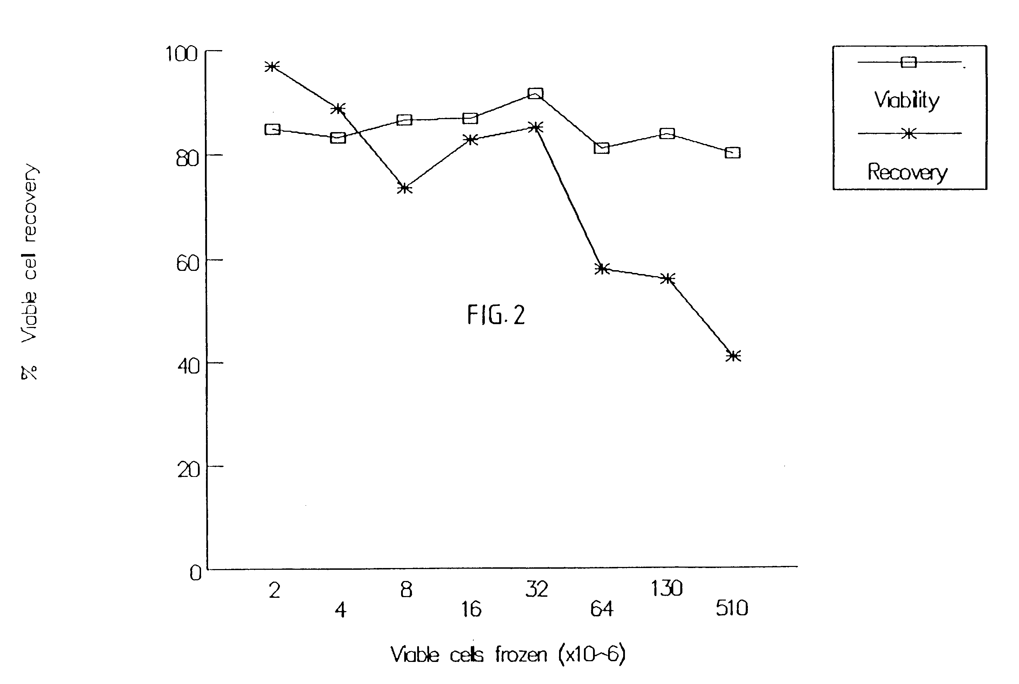

The results, as shown in Figure 2, were that all of the thawed cell suspensions, including that derived from the pellet, had high viabilities. However, examination of the percent viable cell recovery showed that many viable cells had been completely lost from the population. Since there had been no substantial increase in non-viable cells (i.e., cells that stained darkly with trypan blue), the loss of viable cells appeared to occur through a lytic process. Despite this cell loss, excellent viable cell recoveries were obtained for cell suspension frozen at densities as high as $3.2 \times 10^7$ cells/ml (viable cell recovery upon thaw = 84%, or $2.7 \times 10^7$ cells/ml). In order to make certain that cell suspensions derived from the frozen ampules were capable of reestablishing healthy subcultures, flask cultures were generated from each thawed ampule. Excellent subcultures were produced by using cells that had been frozen at densities as high as $1.3 \times 10^8$ cells per ml. In these experiments, the DMSO was not removed after cell thawing. DMSO exhibited toxic effects only when it was present at a concentration of 1.2% (169 mM) or greater in the flask culture.

The freeze/thaw experiment was repeated with an F8 cell population. A series of seven 2-ml ampules was generated by two-fold dilutions of a centrifuged cell pellet. The ampules were frozen for seven days in MOAB medium medium containing 10% DMSO. Data from the thawed cell suspensions showed viabilities to be in the 70-85% range and viable cell recoveries to be in the 70% range or better, i.e. comparable to the results obtained with the BC cells. The most concentrated ampule in this series was seeded at a concentration of $4.5 \times 10^7$ cells/ml and had a recovery rate of 73%, yielding $3.3 \times 10^7$ viable cells per ml

EP 0 394 788 B1

with a viability of 73%.

A duplicate series of 7 ampules was generated with F8 cells, but this time 10% glycerol was used as the cryoprotectant. The results showed that most of the ampules had a thawed cell viability of approximately 80% but a viable cell recovery rate of only 40-50% over most of the density range used for freezing. The exceptions to this were at the most dilute and, surprisingly, most concentrated cell densities. For example, the densest ampule seeded, at $3.5 \times 10^7$ cells per ml, had a recovery rate of 80% and yielded $2.8 \times 10^7$ viable cells per ml at 77% viability. A portion of the thawed cell suspension from this ampule was used to inoculate a roller bottle culture at an inoculation density of $8.4 \times 10^4$ viable cells per ml for a cultivation control. These F8 cells were passaged twice in serum-free medium and achieved 3.16 cumulative population doublings in eight days. The final roller bottle density was $5.3 \times 10^5$ viable cells per ml at a viability of 86%. The glycerol concentration varied from 0.03% in the first passage to 0.02% in the second passage (overall dilution factor = 500x from cryopreserved material).

On the basis of the results from the first three experiments, DMSO was selected as the cryoprotectant of choice for all subsequent experiments. In addition, it was decided that $3 \times 10^7$ cells/ml would be the target cell density in the freezing suspensions.

6. Examples

Example 1 - Experiments in 100-ml Bone Marrow Bags

The first attempts to scale-up the freezing procedure were made with 100-ml bone marrow freezing bags from Stericon. The results of four experiments with single bags are summarized in Table I.

TABLE I

| EXPERIMENTS IN 100-ML BAGS | | | | | | | | |
|---|---|---|---|---|---|---|---|---|
| EXPT NO. | CELL LINE | CELLS | FROZEN | DAYS STORED | CELLS | THAWED | RECOVERIES | |
| | | CELL # | %VIAB | | CELL # | %VIAB | %VCR[1] | RATIO[2] |
| FR-5 | BC | $1.11 \times 10^7$ | 93.1 | 5 | $7.97 \times 10^6$ | 83.9 | 71.8 | .901 |
| FR-7 | BC | $3.84 \times 10^7$ | 94.2 | 8 | $4.13 \times 10^7$ | 92.2 | 107 | .979 |
| FR-8[3] | BC | $1.47 \times 10^7$ | 84.4 | 2 | $1.15 \times 10^7$ | 67.6 | 78.2 | .801 |
| FR-6 | F8 | $4.49 \times 10^7$ | 80.5 | 3 | $1.15 \times 10^7$ | 16.9 | 25.6 | .210 |

Footnotes

[1]%vcr - percent viable cell recovery.

[2]ratio - ratio of viabilities of cells thawed vs. cells frozen.

[3]A controlled-rate freezing device was used in this experiment to freeze the first of the two bags represented here. The recoveries and viability ratios were slightly better for the bag frozen in liquid nitrogen vapor without controlled-rate freezing.

Other abbreviations used: days stored - number of days cells viability.stored in liquid nitrogen or -70°C; %viab - percent viability.

Efforts to freeze and reconstitute BC cells in both high densities and high volumes were successful. The three experiments with BC that are shown in the table involved the use of a number of different techniques for collecting, concentrating, distributing, and freezing the cells. Nevertheless, the viable cell recoveries and the viability ratios are all extremely high. In the case of Expt FR-7 a recovery rate of nearly 100% was obtained when $3.8 \times 10^7$ cells per ml were frozen down.

In contrast to the ease with which the MOAB cell line was scaled-up to 100 ml, the freezing of F8 cells encountered difficulties. Expt. FR-6 (Table I) yielded only a 26% recovery and a 0.210 viability ratio. This appeared to be the consequence of an uncontrolled pH drop to pH 5.8 in the cell suspension during processing prior to freezing. Subsequent experiments employed procedures to prevent such drops, such as incubations at 4°C and shortened processing steps.

Example 2 - Experiments in 500-ml Platelet Freezing Bags

The next step in the scale-up of this procedure was the use of freezing bags with a 500-ml freezing capacity. At this point, the logistics of cell collection and concentration became critical, due to the large number of cells involved. Hollow-fiber membrane filter units, which had been successful in earlier experiments, failed twice at high scale due to clogging and fiber breakage. One experiment (FR-11) was successful, although the suspension generated was not as dense as desired due to filter clogging.

An alternate approach was the use of a high-speed continuous centrifugation device (Haemonetics). Data from three experiments are given in Table II.

TABLE II

| EXPT NO. | CELL LINE | CELLS FROZEN | | DAYS STORED | CELLS THAWED | | RECOVERIES | | SEPARATION |
|---|---|---|---|---|---|---|---|---|---|
| | | CELL # | %VIAB | | CELL # | %VIAB | %VCR | RATIO | |
| FR-11 | F8 | $9.80 \times 10^6$ | 66.7 | 1 | $1.35 \times 10^7$ | 76.7 | 138 | 1.150 | Plasmapur Hollow Fiber |
| FR-10 | F8 | $1.36 \times 10^7$ | 58.7 | 99 | $2.35 \times 10^7$ | 69.1 | 173[1] | 1.179 | Continuous Centrifugation |
| FR-14 | F8 | $9.98 \times 10^6$ | 57.0 | 8 | $8.90 \times 10^6$ | 60.0 | 89.2 | 1.052 | Continuous Centrifugation |

Abbreviations used:

%vcr - percent viable cell recovery.

ratio - ratio of viabilities of cells thawed vs. cells frozen.

days stord - number of days cells stored in liquid nitrogen or -70°C.

%viab - percent viability.

Footnotes
[1]The cell counts made on the frozen and thawed cell populations for this experiment were done by different individuals.

In one experiment, 100% DMSO had been added directly to the cell suspension prior to centrifugation. The result was a flocculent cell concentrate with low viability. The next two experiments FR-10, FR-11, involved the use of 36-50% DMSO added to the concentrate after the centrifugation step. The resultant viable cell recoveries and viability ratios were relatively high. A cultivation test was performed on the thawed cell suspension from Expt. FR-14: a roller bottle was inoculated with $1 \times 10^5$ viable cells per ml and grown in MOAB medium at 37°C. The cells achieved 3.7 population doublings within 5 days and a final culture viability of 90%.

Example 3 - Inoculation of Fermenters

Three 15-L fermenters were inoculated with cells from thawed 500-ml bags. The bags were prepared as set forth in Section 2. In each of the three experiments, a single bag was used to inoculate 8 to 12 L of fermenter suspension at approximately $1 \times 10^6$ viable cells per ml. In experiments 1 and 2, the cell bag was thawed, and a spiked tubing line was used to pump the cell suspension into a holding vessel (3L spinner flask), where the cells were slowly diluted with additional complete medium. Special care was taken when making this dilution so as to avoid osmotic shock due to exit of DMSO frcm the thawed, diluted cells. A two-stage dilution scheme was used: (1) 500 ml of MOAB complete medium was added to 500 ml of cells over a ten-minute period; (2) the resultant 1000 ml of suspension was further diluted by an additional 1000 ml of MOAB medium over a second ten-minute period. The 2 L of cells were recirculated within the apparatus for 5 minutes, and a sample was taken and counted. The inoculum size was calculated and the fermenter was then seeded. An inoculation density of $1 \times 10^6$ viable cells per ml was selected because it is known F8 cells can be grown directly in PM at this density. The entire seeding procedure, from thawing to inoculation required 50 minutes.

The third experiment involved direct seeding of a 15-L fermenter with a thawed bag. Cell dilution took place inside the fermenter vessel itself. This simple approach was deemed suitable for future work under fermenter production conditions. After inoculation, standard fermenter procedures were used for the balance of all three runs.

Details are as follows:

## 1. Fermenter Run F87-FR11

Cell bag FR-11-2 (F8 cells, 500 ml) had been frozen at -70°C, maintained at that temperature for 24 hours, and then thawed rapidly at 37°C. The resultant cell suspension contained $1.4 \times 10^7$ viable cells per ml at a viability of 77%. The cells were diluted with PM and used to inoculate a 15-L Chemap fermenter with 8 L of a seeding suspension at a concentration of $8.4 \times 10^5$ viable cells per ml. The DMSO concentration in the 8-L culture was 0.5% (88 mM). To our knowledge, this was the first fermenter directly inoculated by a cryopreserved cell concentrate. The cells showed exponential growth from Day 1 in culture through the 20 day experiment, expanding from $7.9 \times 10^5$ viable cells per ml to $6.3 \times 10^6$ Viability ranged over the 20 days from 70-70%. rF.VIII titers increased exponentially.

## 2. Fermenter Run F88-B1

Cell bag FR10-2 (F8 cells, 500 ml) had been frozen at -70C, and was maintained at that temperature for 99 days. Upon thawing at 37°C, the resultant cell suspension contained $2.3 \times 10^7$ viable cells per ml at 69% viability. The cells were diluted with MOAB medium and used to inoculate a 15-L Virtis fermenter with 12 L of a seeding suspension at a concentration of $9.8 \times 10^5$ viable cells per ml. The DMSO concentration in this initial suspension was 0.42% (59 mM). After 4 days of culture, the working volume was raised to 15 L and the first dialysis tank was installed, at which time the DMSO concentration equilibrated to 0.03% (4.5 mM). The cells showed exponential growth from Day 3 in culture through the 13 day experiment, expanding from $1 \times 10^6$ cells per ml to $3.2 \times 10^7$ viable cells/ml. Viabilities were generally 80-90% rFVIII titers increased through day 12.

## 3. Fermenter Run F88-2MW

Cell bag FR16-9 (F8 cells, 500 ml) was frozen in liquid nitrogen vapor and stored for 7 days. Upon thawing at 37°C, the cell suspension contained $1.3 \times 10^7$ viable cells per ml at 92% viability. The cells were drained directly into a 15-L Virtis fermenter containing PM medium; the resultant inoculum was 8 L in volume at a concentration of $8 \times 10^5$ viable cells per ml. Dialysis was employed at the start of the fermentation and so the starting concentration of DMSO in the fermenter was 0.03% (4.9 mM).

The cells showed exponential cell growth from Day 2 through the 8 day experiment. Most of the daily viability measurements taken were greater than 90%. rFVIII titers increased exponentially through day 6.

All three of the above fermenter runs produced cell growth, viability, and Factor VIII productivity as good as, if not better than, that obtained by the use of inocula derived by the standard seed-train method. The bag procedure appeared to result in an acceleration of productivity due to earlier attainment of production cell densities and pre-adaptation of the cells to serum-free growth conditions.

Thus there has been provided means and method for the ease of fermenter inoculation due to shortened growth and scale-up time.

A 15-L fermenter can be inoculated with one frozen bag, thereby eliminating weeks of effort and cost involved with the normal seed-tain method. An optimally prepared cell bag will contain 500 ml at a concentration of $3 \times 10^7$ cells per ml, for a total of $1.5 \times 10^{10}$ cells. A typical roller bottle culture of F8 cells contains 500 ml of cells at $5 \times 10^5$, or a total of $2.5 \times 10^8$ cells. A single frozen cell bag is thus equivalent to 60 roller bottles of cells, but does not require the laborious effort expended when roller bottles are used for inoculation. This simplification of the inoculation process also greatly reduces the chances of contamination during seeding.

Furthermore, the use of frozen cell bags make it easier to plan and maintain production schedules. Any loss of fermentation capacity due to contamination or other technical failures can be rapidly corrected by thawing other bags. There is no loss in laboratory efficiency due to waiting time for the new cell seed.

**Claims**

**1.** A method of preparing mammalian cells for use for the direct inoculation of fermenters, comprising:

    (a) culturing the cells in a first fermenter;

    (b) separating the cells from step (a) in aliquots of at least 100 ml at a density of at least $10^6$ cells per ml;

(c) freezing the cells from step (b) to at least -70°C in flexible film containers containing a cryopreservative solution; and

(d) thawing the cells from step (c) for inoculation into a second fermenter, said cells having at least 70% viability.

2. The method of claim 1 wherein said cells are immortal.

3. The method of claim 1 wherein said aliquots are separated into and frozen in 500 ml EVA bags.

4. The method of claim 1 wherein said freezing is to -196°C.

5. The method of claim 1 wherein said culturing in the first fermenter is in the same medium as is used in the second fermenter.

6. The method of claim 1 wherein said cryopreservative is added as a dilute solution of not more than 50% DMSO.

7. The method of claim 6 wherein said cryopreservative further comprises 1-2% plasma protein fraction.

8. A method of inoculating a fermenter with immortalized mammalian cells comprising:

(a) preparing a frozen, flexible film container containing at least 100 ml of a cell culture having at least $10^6$ cells per ml, 5-15 % DMSO, and tissue culture media;

(b) holding said container at -196°C until needed; and

(c) inoculating said fermenter with said containers, thereby producing an initial fermenter density of $10^5$ - $10^7$ cells per ml, said cells being at least 70% viable on inoculation.

9. A cell culture system for large scale inoculation of a fermenter, comprising:

(a) a flexible film container of at least 500 ml capacity;

(b) at least 100 ml of frozen tissue culture media in said container, the media comprising 5 - 15% DMSO cryopreservative; and

(c) at least 100 ml of immortal mammalian cells in said media at a density of at least $10^6$ cells per ml and having at least 70% viability.

10. The cell culture system of claim 9 wherein said media contains no serum.

11. The cell culture system of claim 9 wherein said flexible film is ethylene vinyl acetate.

**Patentansprüche**

1. Verfahren zur Herstellung von Säugetierzellen zum Einsatz für die direkte Inokulation von Fermentern, umfassend:

(a) Züchten der Zellen in einem ersten Fermenter;

(b) Abtrennen der Zellen von Stufe (a) in Aliquotmengen von mindestens 100 ml bei einer Dichte von mindestens $10^6$ Zellen pro ml;

(c) Einfrieren der Zellen von Stufe (b) bei mindestens -70°C in flexiblen Filmbehältern mit einem Gehalt an einer Kryokonservierungslösung; und

(d) Auftauen der Zellen von Stufe (c) für die Inokulation in einen zweiten Fermenter, wobei diese Zellen eine Lebensfähigkeit von mindestens 70 % aufweisen.

2. Verfahren nach Anspruch 1, wobei die Zellen unsterblich sind.

3. Verfahren nach Anspruch 1, wobei die Aliquotmengen zur Auftrennung in 500 ml EVA-Beutel gegeben und dort eingefroren werden.

4. Verfahren nach Anspruch 1, wobei das Einfrieren bis zu einer Temperatur von -196°C erfolgt.

5. Verfahren nach Anspruch 1, wobei die Züchtung im ersten Fermenter im gleichen Medium, wie es im zweiten Fermenter verwendet wird, durchgeführt wird.

**6.** Verfahren nach Anspruch 1, wobei das Kryokonservierungsmittel als eine verdünnte Lösung von nicht mehr als 50 % DMSO zugegeben wird.

**7.** Verfahren nach Anspruch 6, wobei das Kryokonservierungsmittel ferner 1-2 % Plasmaproteinfraktion umfaßt.

**8.** Verfahren zum Inokulieren eines Fermenters mit immortalisierten Säugerzellen, umfassend:
(a) Herstellen eines gefrorenen, flexiblen Filmbehälters mit einem Gehalt an mindestens 100 ml einer Zellkultur mit mindestens $10^6$ Zellen pro ml, 5-15 % DMSO und Gewebekulturmedien;
(b) Aufbewahren des Behälters bei -196°C bis zum Bedarf; und
(c) Inokulieren des Fermenters mit den Behältern, wodurch eine anfängliche Fermenterdichte von $10^5$ - $10^7$ Zellen pro ml erzeugt wird, wobei die Zellen bei der Inokulation zu mindestens 70 % lebensfähig sind.

**9.** Zellkultursystem für die großtechnische Inokulation eines Fermenters, umfassend:
(a) einen flexiblen Filmbehälter mit einem Fassungsvermögen von mindestens 500 ml;
(b) mindestens 100 ml gefrorenes Gewebekulturmedium im Behälter, wobei das Medium 5-15 % DMSO-Kryokonservierungsmittel enthält; und
(c) mindestens 100 ml unsterbliche Säugerzellen im Medium in einer Dichte von mindestens $10^6$ Zellen pro ml und einer Lebensfähigkeit von mindestens 70 %.

**10.** Zellkultursystem nach Anspruch 9, wobei das Medium kein Serum enthält.

**11.** Zellkultursystem nach Anspruch 9, wobei der flexible Film aus Ethylen-Vinylacetat ist.

**Revendications**

**1.** Procédé de préparation de cellules de mammifère destinées à être utilisées pour l'ensemencement direct de fermenteurs, comprenant :
(a) la culture des cellules dans un premier fermenteur ;
(b) la séparation des cellules de l'étape (a) par portions aliquotes d'au moins 100 ml, à une densité d'au moins $10^6$ cellules par ml ;
(c) la congélation des cellules de l'étape (b) à au moins -70°C dans des récipients constitués de films flexibles, contenant une solution d'un cryoconservateur ; et
(d) la décongélation des cellules de l'étape (c) pour l'ensemencement d'un second fermenteur, lesdites cellules possédant une viabilité d'au moins 70 %.

**2.** Procédé suivant la revendication 1, dans lequel les cellules sont immortelles.

**3.** Procédé suivant la revendication 1, dans lequel les portions aliquotes sont séparées et congelées dans des sachets de 500 ml en EVA.

**4.** Procédé suivant la revendication 1, dans lequel la congélation est effectuée à -196°C.

**5.** Procédé suivant la revendication 1, dans lequel la culture dans le premier fermenteur est effectuée dans le même milieu que celui utilisé dans le second fermenteur.

**6.** Procédé suivant la revendication 1, dans lequel le cryoconservateur est ajouté sous forme d'une solution diluée ne renfermant pas plus de 50 % de DMSO.

**7.** Procédé suivant la revendication 6, dans lequel le cryoconservateur comprend en outre 1 à 2 % de fraction de protéines plasmatiques.

**8.** Procédé pour ensemencer un fermenteur avec des cellules immortalisées de mammifère comprenant :
(a) la préparation d'un récipient constitué d'un film flexible, congelé, contenant au moins 100 ml d'une culture de cellules renfermant au moins $10^6$ cellules par ml, 5 à 15 % de DMSO et un milieu de culture de tissus ;
(b) le maintien dudit récipient à -196°C jusqu'à son utilisation ; et

(c) l'ensemencement dudit fermenteur avec de tels récipients, ce qui permet de parvenir à une densité initiale dans le fermenteur d'au moins $10^5$-$10^7$ cellules par ml, lesdites cellules présentant une viabilité d'au moins 70 % lors de l'ensemencement.

9. Dispositif de culture de cellules pour un ensemencement à grande échelle d'un fermenteur, comprenant :
(a) un récipient constitué d'un film flexible, d'une capacité d'au moins 500 ml ;
(b) au moins 100 ml d'un milieu congelé pour culture de tissus dans ledit récipient, le milieu comprenant 5 à 15 % de DMSO servant de cryoconservateur ; et
(c) au moins 100 ml de cellules immortelles de mammifère dans ledit milieu, à une densité d'au moins $10^6$ cellules par ml, et possédant une viabilité d'au moins 70 %.

10. Dispositif de culture de cellules suivant la revendication 9, dans lequel le milieu ne contient pas de sérum.

11. Dispositif de culture de cellules suivant la revendication 9, dans lequel le film flexible consiste en un film d'éthylène-acétate de vinyle.

# FERMENTER INOCULATION METHODS

FIG.1

FIG. 2

Viable cell recovery %

Viable cells frozen (x10~6)

Viability

Recovery

EP 0 394 788 B1